# EUROPEAN PATENT APPLICATION

(11) **EP 2 196 151 A1**
(43) Date of publication of application: **16.06.2010**
(21) Application number: 09178885.1
(22) Date of filing: 11.12.2009
(51) Int. Cl.: A61B 8/00, G01S 15/89

(54) **Ultrasound system and method for forming a plurality of three-dimensional ultrasound images**

(30) Priority: 15.12.2008 KR 20080127090
(71) Applicant: MEDISON CO., LTD., Kangwon-do 250-870 (KR)
(72) Inventor: Kim, Sung Yoon, 135-851, Seoul (KR); Lee, Suk Jin, 135-851, Seoul (KR); Choi, Do Young, 135-851, Seoul (KR)
(74) Representative: Schmid, Wolfgang

(57) **Abstract**

The present invention relates to an ultrasound system and method capable of forming a plurality of three-dimensional ultrasound images at the time. The ultrasound system comprises: a volume data acquisition unit configured to transmit ultrasound signals to a target object, receive ultrasound echo signals reflected from the target object and acquire ultrasound data based on the received ultrasound echo signals, the volume data acquisition unit being further configured to form a plurality of sets of volume data based on the ultrasound data; and a processor configured to render the plurality of sets of the volume data to thereby form a plurality of three-dimensional ultrasound images.

## Description

### CROSS-REFERENCE TO RELATED APPLICATIONS

The present application claims priority from Korean Patent Application No. 10-2008-0127090 filed on December 15, 2008, the entire subject matter of which is incorporated herein by reference.

### TECHNICAL FIELD

The present invention generally relates to ultrasound systems, and more particularly to an ultrasound system and method for forming a plurality of three-dimensional ultrasound images.

### BACKGROUND

The ultrasound system has become an important and popular diagnostic tool due to its non-invasive and non-destructive nature. Modern high-performance ultrasound imaging diagnostic systems and techniques are commonly used to produce two or three-dimensional images of internal features of patients (target objects).

Generally, the ultrasound system may provide the three-dimensional ultrasound image including clinical information such as spatial information and anatomical figures of the target objects, which cannot be provided through the two-dimensional ultrasound image. The ultrasound system may transmit ultrasound signals to the target objects, receive ultrasound echo signals reflected from the target objects and form volume data based on the ultrasound echo signals. The ultrasound system may further form the three-dimensional ultrasound image including the clinical information by rendering the volume data.

Generally, the conventional ultrasound system forms only one set of volume data and renders the volume data set to thereby form a three-dimensional ultrasound image. That is, the conventional ultrasound system does not provide a function of performing rendering upon at least two or more sets of volume data. Further, a plurality of three-dimensional ultrasound images indicative of the same or different objects cannot be provided at the same time.

### SUMMARY

An embodiment for forming a plurality of three-dimensional ultrasound images is disclosed herein. In one embodiment, by way of non-limiting example, a system for forming the plurality of three-dimensional ultrasound images, comprises: a volume data acquisition unit configured to transmit ultrasound signals to a target object, receive ultrasound echo signals reflected from the target object and acquire ultrasound data based on the received ultrasound echo signals, the volume data acquisition unit being further configured to form a plurality of sets of volume data based on the ultrasound data; and a processor configured to render the plurality of sets of the volume data to thereby form a plurality of three-dimensional ultrasound images.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

- FIG. 1: is a block diagram showing an illustrative embodiment of an ultrasound system.
- FIG. 2: is a block diagram showing a volume data acquisition unit located within the ultrasound system.
- FIG. 3: is a block diagram showing an illustrative embodiment of a processor.

### DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

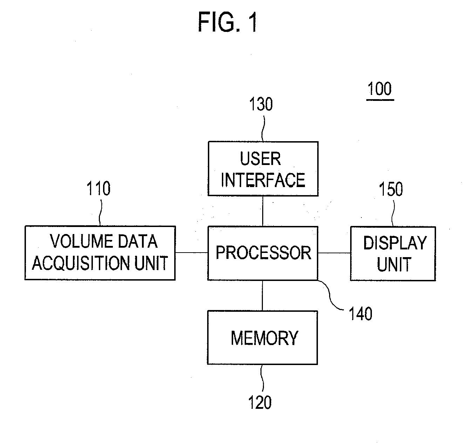

FIG. 1 is a bock diagram showing an illustrative embodiment of an ultrasound system. The ultrasound system 100 may include a volume data acquisition unit 110, a memory 120, a user interface 130, a processor 140 and a display unit 150.

The volume data acquisition unit 110 may be configured to transmit ultrasound signals to a target object (not shown) and receive ultrasound echo signals reflected from the target object. The volume data acquisition unit 110 may be further configured to acquire ultrasound data based on the received ultrasound echo signals to thereby form the volume data.

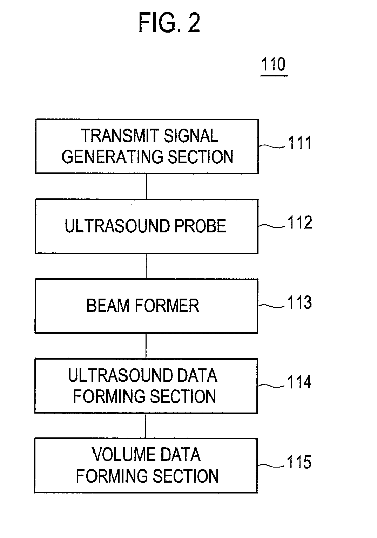

FIG. 2 is a block diagram showing the volume data acquisition unit 110 provided within the ultrasound system. The ultrasound data acquisition unit 110 may include a transmit (Tx) signal generating section 111, an ultrasound probe 112 including a plurality of transducer elements (not shown), a beam former 113, an ultrasound data forming section 114 and a volume data forming section 115.

The Tx signal generating section 111 may generate Tx signals according to an image mode set in the ultrasound system 100. The image mode may include a brightness (B) mode, a Doppler (D) mode, a color flow mode, etc. In one exemplary embodiment, the B mode may be set in the ultrasound system 100 to obtain a B mode ultrasound image.

The ultrasound probe 112 may receive the Tx signals from the Tx signal generating section 111 and generate ultrasound signals, which may travel into the target object. The ultrasound probe 112 may further receive ultrasound echo signals reflected from the target object and convert them into electrical receive signals. In such a case, the electrical receive signals may be analog signals. The ultrasound probe 112 may be a three-dimensional probe, a two-dimensional probe, a one-dimensional probe or the like.

The beam former 113 may convert the electrical receive signals outputted from the ultrasound probe 112 into digital signals. The beam former 113 may further apply delays to the digital signals in consideration of the distances between the transducer elements and focal points to thereby output receive-focused signals.

The ultrasound data forming section 114 may form a plurality of ultrasound data by using the receive-focused signals. In one embodiment, the plurality of ultrasound data may be radio frequency (RF) data or IQ data. The volume data forming section 115 may form a plurality of sets of volume data based on the ultrasound data.

Referring back to FIG. 1, the memory 120 may store the plurality of sets of the volume data. In one embodiment, by way of non-limiting examples, the memory 120 may include at least one of a random access memory (RAM), a hard disk drive or the like.

The user interface 130 may be operable to allow a user to input user instructions. In one embodiment, by way of non-limiting examples, the user interface 130 may include at least one of a control panel (not shown), a keyboard (not shown), a mouse (not shown) or the like. The user instructions may include first to eighth user instructions. The first user instruction may be an instruction to select at least two sets of volume data among the plurality of sets of the volume data stored in the memory 130. The second user instruction may be an instruction to set geometries of the selected sets of the volume data. In one embodiment, the geometry may include rotation, translation and zoom of the volume data. The geometry may be identically set to the selected sets of the volume data. The third user instruction may be an instruction to select one of rendering methods of performing rendering upon the selected sets of the volume data or the sets of the volume data having the set geometry. The rendering methods may include a ray-casting projection method, a maximum projection method, a minimum projection method, an average projection method, etc. The fourth user instruction may be an instruction to select colors representing the respective three-dimensional ultrasound images. The fifth user instruction may be an instruction to compound the plurality of three-dimensional ultrasound images. The sixth user instruction may be an instruction to select an application corresponding to the type of an examining portion of the target object. The seventh user instruction may be an instruction to select a diagnosis mode of the ultrasound system 100. The eighth user instruction may be an instruction to display the three-dimensional ultrasound images.

The processor 140 may be operable to render the selected sets of volume data to thereby form a plurality of three-dimensional ultrasound images. Moreover, the processor 120 may be further operable to compound the plurality of three-dimensional ultrasound images to thereby form a compound image. In one embodiment, by way of non-limiting examples, the processor 150 may include at least one of a central processing unit (CPU), a graphic processing unit (GPU) and the like.

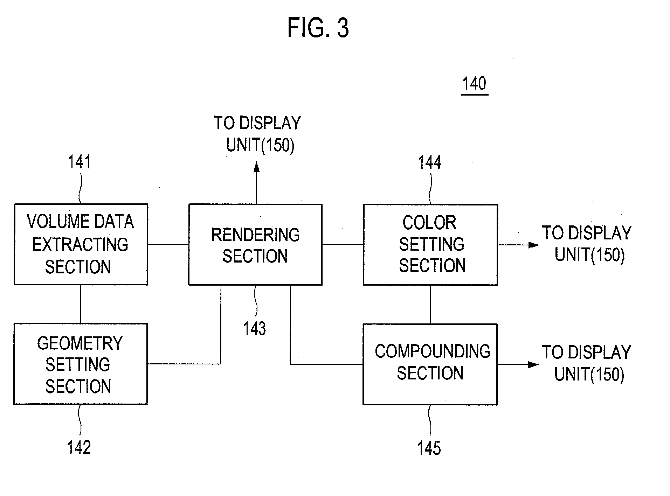

FIG. 3 is a block diagram showing an illustrative embodiment of the processor 140. The processor 140 may include a volume data extracting section 141, a geometry setting section 142, a rendering section 143, a color setting section 144 and a compounding section 145. The processor 140 may further include a voxel interval regulating section (not shown), which may be operable to regulate voxel intervals in the plurality of volume data for accurate measurement.

The volume data extracting section 141 may be operable to extract the at least two or more sets of the volume data, which are selected in response to the first user instruction, from the memory 120. The geometry setting section 142 may set the geometry of the extracted sets of volume data responsive to the second user instruction. In one embodiment, assuming that first to fourth sets of the volume data are extracted, the geometry setting section 142 may set the rotation, translation and zoom of each of the first to fourth sets of the volume data. In one embodiment, by way of non-limiting example, the geometry setting section 142 may identically set the rotation, translation and zoom to the first to fourth sets of the volume data.

The rendering section 143 may render the extracted sets of the volume data or the volume data having the set geometry responsive to the third user instruction to thereby form the plurality of ultrasound images. In one embodiment, by way of non-limiting example, the rendering section 143 may render the first set of the volume data by using the ray-casting projection method to form the three-dimensional ultrasound image. The rendering section 143 may render the second set of the volume data by using the ray-casting projection method to form the three-dimensional ultrasound image. The rendering section 143 may render the third set of the volume data by using the ray-casting projection method to form the three-dimensional ultrasound image. It may render the fourth set of the volume data by using the ray-casting projection method to form the three-dimensional ultrasound image. The first to fourth sets of the volume data may be the extracted sets of the volume data or the sets of the volume data having the set geometry. In one embodiment, the rendering section 143 may use an identical rendering method to perform rendering upon the first to fourth sets of the volume data.

The color setting section 144 may be operable to set colors corresponding to the respective three-dimensional ultrasound images formed at the rendering section 143 responsive to the fourth user instruction. The compounding section 145 may compound the plurality of three-dimensional ultrasound images formed by the rendering section 143 or the plurality of three-dimensional ultrasound images having the set colors to thereby form the compound image responsive to the fifth user instruction. Moreover, the compounding section 145 may set weights for adjusting transparency of the respective three-dimensional ultrasound images. The compounding section 145 may compound the three-dimensional ultrasound image with the transparency adjusted to thereby form the compound image.

The display unit 150 may display the plurality of three-dimensional ultrasound images, which are formed at the rendering unit 143 of the processor 140, at the same time. The display unit 150 may display the three-dimensional ultrasound images having the set colors at the same time. The display unit 150 may display the compound image formed by the compounding section 145. Moreover, the display unit 150 may display the plurality of three-dimensional ultrasound images and the compound image at the same time.

In another embodiment, the present invention may provide a computer readable medium, comprising instructions that, when executed by a processor performs a method of forming a plurality of three-dimensional ultrasound images in the ultrasound system, cause the processor to perform steps comprising: obtaining a plurality of ultrasound data, forming at least two sets of volume data based on the plurality of ultrasound data and forming a plurality of three-dimensional ultrasound images by rendering the at least two sets of the volume data. The computer readable medium may include a floppy disk, a hard disk, a memory, a digital versatile disc or the like.

Any reference in this specification to "one embodiment," "an embodiment," "example embodiment," "illustrative embodiment," etc. means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to affect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
a volume data acquisition unit configured to transmit ultrasound signals to a target object, receive ultrasound echo signals reflected from the target object and acquire ultrasound data based on the received ultrasound echo signals, the volume data acquisition unit being further configured to form a plurality of sets of volume data based on the ultrasound data; and
a processor configured to render the plurality of sets of the volume data to thereby form a plurality of three-dimensional ultrasound images.

2. The ultrasound system of Claim 1, further comprising:
a user interface configured to receive a first user instruction for selecting at least two sets of the volume data among the plurality of sets of the volume data.

3. The ultrasound system of Claim 2, wherein the user interface is further configured to receive a second user instruction for setting geometries of the at least two sets of the volume data, a third user instruction for selecting one of rendering methods for performing rendering upon the selected sets of the volume data or the sets of the volume data having the set geometries, a fourth user instruction for selecting colors presented on the three-dimensional ultrasound images and a fifth user instruction for compounding the three-dimensional ultrasound images.

4. The ultrasound system of Claim 3, wherein the processor comprises:
a volume data extracting section configured to extract the at least two sets of the volume data responsive to the first user instruction;
a geometry setting section configured to set the geometries of the at least two sets of the volume data responsive to the second user instruction;
a rendering section configured to form the three-dimensional ultrasound images by rendering the at least two sets of the volume data having the set geometries responsive to the third user instruction;
a color setting section configured to set the colors of the three-dimensional ultrasound images responsive to the fourth user instruction; and
a compounding section configured to compound the three-dimensional ultrasound images formed by the rendering section responsive to the fifth user instruction.

5. The ultrasound system of Claim 4, wherein the geometries include rotation, translation and zoom of the at least two sets of the volume data.

6. A method of forming three-dimensional ultrasound images in an ultrasound system comprising:
a) obtaining a plurality of ultrasound data;
b) forming a plurality of sets of volume data based on the plurality of ultrasound data; and
c) forming a plurality of three-dimensional ultrasound images by rendering the plurality of sets of the volume data.

7. The method of Claim 6, wherein the step c) comprises:
c1) receiving a first user instruction for selecting the at least two sets of the volume data;
c2) extracting the at least two sets of the volume data responsive to the first user instruction;
c3) receiving a second user instruction for selecting one of rendering methods to render the at least two sets of the volume data; and
c4) forming the three-dimensional ultrasound images by rendering the at least two sets of the volume data using the selected rendering method.

8. The method of Claim 6, wherein the step c) comprises:
c1) receiving a first user instruction for selecting the at least two sets of the volume data;
c2) extracting the at least two sets of the volume data responsive to the first user instruction;
c3) receiving a second user instruction for setting geometries of the at least two sets of the volume data, wherein the geometries include rotation, translation and
zoom of the at least two sets of the volume data;
c4) setting the geometries of the at least two sets of the volume data responsive to the second user instruction;
c5) receiving a third user instruction for selecting one of rendering methods to render the at least two sets of the volume data having the set geometries; and
c6) forming the three-dimensional ultrasound images by rendering at least two sets of the volume data having the set geometries responsive to the third user instruction using the selected rendering method.

9. The method of Claim 8, further comprising:
c7) receiving a fourth user instruction for selecting colors of the three-dimensional ultrasound images;
c8) setting the selected colors to the three-dimensional ultrasound images;
c9) receiving a fifth user instruction for compounding the three-dimensional ultrasound images having the set colors; and
c10) compounding the three-dimensional ultrasound images having the set colors responsive to the fifth user instruction.

10. A computer readable medium comprising instructions that, when executed by a processor, perform a method of forming three-dimensional ultrasound images, comprising steps of:
a) obtaining a plurality of ultrasound data;
b) forming at least two sets of volume data based on the plurality of ultrasound data; and
c) forming a plurality of three-dimensional ultrasound images by rendering the at least two sets of the volume data.

11. The computer readable medium of Claim 10, wherein the step c) comprises:
c1) receiving a first user instruction for selecting the at least two sets of the volume data;
c2) abstracting the at least two sets of the volume data responsive to the first user instruction;
c3) receiving a second user instruction for selecting one of rendering methods to render the at least two sets of the volume data; and
c4) forming the three-dimensional ultrasound images by rendering the at least two sets of the volume data using the selected rendering method.

12. The computer readable medium of Claim 10, wherein the step c) comprises:
c1) receiving a first user instruction for selecting the at least two sets of the volume data;
c2) extracting the at least two sets of the volume data responsive to the first user instruction;
c3) receiving a second user instruction for setting geometries of the at least two sets of the volume data, wherein the geometries include rotation, translation and
zoom of the at least two sets of the volume data;
c4) setting the geometries of the at least two sets of the volume data responsive to the second user instruction;
c5) receiving a third user instruction for selecting one of rendering methods to render the at least two sets of the volume data; and
c6) forming the three-dimensional ultrasound images by rendering at least two sets of the volume data having the set geometries responsive to the third user instruction using the selected rendering method.

13. The computer readable medium of Claim 12, wherein the steps further comprises:
c7) receiving a fourth user instruction for selecting colors of the three-dimensional ultrasound images;
c8) setting the selected colors to the three-dimensional ultrasound images;
c9) receiving a fifth user instruction for compounding the three-dimensional ultrasound images having the set colors; and
c10) compounding the three-dimensional ultrasound images having the set colors responsive to the fifth user instruction.
